# EUROPÄISCHE PATENTANMELDUNG

(11) **EP 3 299 804 A1**
(43) Veröffentlichungstag der Anmeldung: **28.03.2018**
(21) Anmeldenummer: 16190831.4
(22) Anmeldetag: 27.09.2016
(51) Int. Cl.: G01N 27/02, G01N 27/07, G01N 33/18, G01N 33/487

(54) **VERFAHREN UND VORRICHTUNG ZUR ANALYSE DER BAKTERIENDICHTE IN TRINKWASSER**

(71) Anmelder: Georg Fischer JRG AG, 4450 Sissach (CH)
(72) Erfinder: Lüscher, Marcel, 5600 Ammerswil (CH); Nacke, Thomas, 37308 Heilbad Heiligenstadt (DE); Schröder, Olaf, 04347 Leipzig (DE)
(74) Vertreter: Fenner, Seraina

(57) **Zusammenfassung**

Die Erfindung betrifft ein Verfahren und eine Vorrichtung zur quantitativen und/oder qualitativen Analyse von zellulären Bestandteilen in Flüssigkeiten, z.B. in Wasser, insbesondere Trinkwasser, z.B. Bakterien oder eukaryontischen Zellen. Die Vorrichtung umfasst eine Messkammer mit einem Elektrodenarray mit einer Gegenelektrode und mehreren sich in Bezug auf ihr Material unterscheidenden Arbeitselektroden sowie einen Impedanzanalysator sowie deren Verwendung in Verfahren zur erfindungsgemäßen Analyse. Es werden auch geeignete Computerprogrammprodukte bereitgestellt.

## Beschreibung

Die Erfindung betrifft ein Verfahren und eine Vorrichtung zur quantitativen und/oder qualitativen Analyse von zellulären Bestandteilen in Flüssigkeiten, z.B. in Wasser, insbesondere Trinkwasser, z.B. Bakterien oder eukaryontischen Zellen. Die Vorrichtung umfasst eine Messkammer mit einem Elektrodenarray mit einer Gegenelektrode und mehrere sich in Bezug auf ihr Material unterscheidenden Arbeitselektroden sowie einen Impedanzanalysator sowie deren Verwendung in Verfahren zur erfindungsgemäßen Analyse. Dazugehörend werden auch geeignete Computerprogrammprodukte bereitgestellt.

Mikrobiologische Belastungen sind ein ernstes Problem der Trinkwasserversorgung. In Wasser, auch in Trinkwasser bilden sich unter bestimmten Umständen Bakterien, die bei Überschreiten einer gesetzlich festgelegten Menge meldepflichtig sind und zu gesundheitlichen Problemen führen können. Infektionen durch mikrobielle Verunreinigungen nehmen weltweit deutlich zu.

Stand der Technik bei der Feststellung einer mikrobiologischen Kontamination ist die Entnahme einer Wasserprobe und eine Untersuchung mittels Kultivierungsmethode. Nachteil dieser Methode ist die dafür erforderliche Kultivierungszeit von mehreren Tagen, ehe ein Nachweis zur Verfügung steht. Eine weitere Analysemethode ist die Untersuchung mittels Durchflusszytometrie (Nachweisgrenze 100 Zellen/ml). Die Durchflusszytometrie steht auf Grund der Komplexität und dem Preis der dafür erforderlichen Geräte zur Zeit nur für Labore zur Verfügung.

Nachteil beider Methoden ist, dass die Analysemethoden nur mit sehr grossem Aufwand onlinetauglich sind und eine Probe nach exakt einzuhaltenden Vorgaben entnommen und diese dem Labor unter Einhaltung von Transportbedingungen übermittelt werden muss. Untersuchungen sind deshalb nicht nur aufwendig, sondern auch stark fehlerbehaftet und teuer. Bisherige Verfahren erlauben also keine Online-Detektion, bzw. eine zeitnahe Vor-Ort-Detektion, von mikrobiellen Verunreinigungen im Trinkwasser.

Elektrische Eigenschaften von Gewebe und Zellsuspensionen sind schon seit Jahrzehnten bekannt (Schwan, H. (1957). "Electrical Proporties of Tissue and Cell Suspensions." Advan. Biol. Med. Phys.(5): 147-205).

Yang, L. J. (2008). "Electrical impedance spectroscopy for detection of bacterial cells in suspensions using interdigitated microelectrodes." Talanta 74(5): 1621-1629, beschreibt, dass unter Verwendung von interdigitierenden Mikroelektroden verschiedene Konzentration bakterieller Zellen in deionisiertem Wasser, nicht aber in Phosphat-gepufferter Salzlösung (PBS), zu unterschiedlichen Impedanzspektren führen können.

Varshney, M. and Y. Li (2009). "Interdigitated Array Microelectrodes based Impedance Biosensors for Detection of Bacterial Cells." Biosensors and Bioelectronics, beschreibt verschiedene im Stand der Technik genutzte Verfahren zur Impedanz-basierten Analyse von Bakterienlösungen.

WO 2009/090280 A1, auch veröffentlicht als EP 2 243 824 A1, zeigt eine Vorrichtung, ein Verfahren und eine Verwendung eines elektronischen Chip-Elements zur Messung einer Biomasse-Konzentration, wobei Änderungen der Impedanz, welche durch die Anwesenheit von Biomasse hervorgerufen werden, mit Hilfe von zwei Elektroden, die in ein Halbleitermaterial in Form eines elektronischen Chips eingebettet sind, gemessen werden sollen. Dies soll z.B. zur Kontrolle von Trinkwasser nützlich sein.

Ausgehend von dem bekannten Stand der Technik haben sich die Erfinder die Aufgabe gestellt, eine vorteilhafte Vorrichtung und ein Verfahren zur Verfügung zu stellen, welches eine sensitive Analyse zellulärer Bestandteile von Flüssigkeiten unter online-Bedingungen erlaubt, insbesondere von bakteriellen Verunreinigungen in Trinkwasser. Diese Aufgabe wird z.B. durch den Gegenstand der Ansprüche gelöst.

Die Erfindung betrifft eine Vorrichtung zur quantitativen und optional qualitativen Analyse von zellulären Bestandteilen einer Flüssigkeit, umfassend (a) eine Messkammer, welche ein Elektrodenarray mit mehreren sich in Bezug auf ihr Material unterscheidenden Arbeitselektroden und einer Gegenelektrode aufweist und (b) einen Impedanzanalysator.

Die Vorrichtung ist bevorzugt zur quantitativen und qualitative Analyse zellulärer Bestandteile einer Flüssigkeit geeignet. Eine qualitative Analyse kann z.B. eine Aussage über die Größe, Form und/oder Art der zellulären Bestandteile erlauben.

Die Flüssigkeit ist im Rahmen der Erfindung bevorzugt eine wässrige Flüssigkeit, z.B. Wasser (wie Trinkwasser, Betriebswasser, entmineralisiertes Wasser, membrangefiltertes Wasser, Oberflächenwasser, Abwasser, etwa vor oder nach Reinigung, Süßwasser, Salzwasser, alkoholfreie oder alkoholhaltige Getränke (z.B. Mineralwasser oder Getränke, die unter Mitwirkung von Mikroorganismen hergestellt werden, wie Bier, Wein, Sake, Bionade), Zwischenprodukte der jeweiligen Getränkeherstellung, Zellkulturmedium oder eine Zellkultur (z.B. eine Kultur von Bakterien oder eukaryontischen Zellen), Bevorzugt handelt es sich um Wasser, am meisten bevorzugt Trinkwasser. Unter Trinkwasser wird gemäß nationalen Bestimmungen zum Trinken durch Menschen vorgesehenes Wasser verstanden, insbesondere Trinkwasser gemäß § 3 TrinkwV (Trinkwasserverordnung).

An zellulären Bestandteilen können insbesondere Mikroorganismen, vor allem Bakterien oder eukaryontische Zellen, z.B. Hefen oder andere eukaryontische Einzeller, aber auch Mehrzeller, analysiert werden. Es kann etwa auch das Vorkommen von Algen, z.B. in Wasser, untersucht werden. Typischerweise wird eine quantitative und optional qualitative Analyse von Bakterien vorgenommen, z.B. von grampositiven und /oder gramnegativen Bakterien, bevorzugt werden aber auch andere zelluläre Bestandteile von der Analyse erfasst. Von besonderem Interesse ist oft die Analyse von potentiell pathogenen zellulären Bestandteilen, insbesondere potentiell pathogenen Bakterien. Als zelluläre Bestandteile einer Flüssigkeit werden intakte, also von einer Membran und/oder Zellwand umhüllte Zellen bezeichnet. Diese weisen ionische und kapazitive Komponenten auf, die eine Detektion über eine Impedanzmessung ermöglichen.

Zu den relevanten Mikroorganismen in Wasser, etwa in Trinkwasser, welche erfindungsgemäß analysiert werden können, gehören umweltassoziierte Mikroorganismen, aquatische Mikroorganismen und fäkale Mikroorganismen, z.B. Clostridium sp., Nitrosomonas sp., Nitrobacter sp., Pseudomonas sp. (z.B. Pseudomonas aeruginosa), Legionella spp. (z.B. Legionella pneumophila), Coliforme Bakterien, wie Citrobacter sp., Enterokokken, Streptokokken, Escherichia sp. (z.B. Escherichia coli), Salmonellen, Yersinien und andere Enterobakterien oder Saccharomyces cerevisiae. Es ist auch möglich, zelluläre Bestandteile in anderen Flüssigkeiten zu detektieren, z.B. erwünschte Mikroorganismen wie Hefen (z.B. Saccharomyces cerevisiae) etwa in Getränken wie Bier oder Zwischenprodukten der Bierherstellung. Auch Getränkeschädlinge können erfindungsgemäß analysiert werden.

Das Elektrodenarray umfasst mehrere sich in Bezug auf ihr Material unterscheidende Arbeitselektroden, mindestens zwei Arbeitselektroden, bevorzugt, mindestens drei, mindestens vier, mindestens fünf, mindestens sechs, mindestens sieben, mindestens acht, mindestens neun, mindestens zehn, mindestens elf oder mindestens zwölf Arbeitselektroden. Je mehr Arbeitselektroden vorgesehen sind, desto genauer wird die Messung und desto zuverlässiger kann eine qualitative und/oder quantitative Aussage über die zellulären Bestandteile der zu analysierenden Flüssigkeit gemacht werden.

Die Arbeitselektroden unterscheiden sich in Bezug auf das Redoxpotential ihrer Oberfläche voneinander. Die Arbeitselektroden der erfindungsgemäßen Vorrichtung weisen eine Oberfläche aus verschiedenen Materialien auf, ausgewählt aus der Gruppe umfassend Au, Ir, Pd, Pt, Fe (insbesondere rostfreier Stahl), Rh und Re. So ist bevorzugt die Oberfläche mindestens einer Arbeitselektrode des Elektrodenarrays, bevorzugt von mindestens zwei, mindestens drei, mindestens vier, mindestens fünf, mindestens sechs oder allen Arbeitselektroden des Arrays aus einem dieser Materialien. Es hat sich herausgestellt, dass besonders gute Ergebnisse mit Arbeitselektroden mit einer Oberfläche aus Ir, Rh und/oder Re oder Materialien vergleichbaren Redoxpotentials erhalten werden. So umfasst ein erfindungsgemäßes Elektrodenarray bevorzugt mindestens Arbeitselektroden mit einer Oberfläche aus Ir, Rh und Re. Bevorzugt umfasst das Elektrodenarray mindestens sechs Arbeitselektroden mit einer Oberfläche aus je einem der Materialien Au, Ir, Pd, Pt, Rh und Re.

Die unterschiedlichen Oberflächenmaterialien haben den technischen Effekt, dass sie zu unterschiedlichen Messdynamiken führen, es ergeben sich also unterschiedliche Spektren der verschiedenen Elektroden bei Messungen der gleichen Probe unter gleichen Bedingungen. Dadurch wird die Aussagekraft der Messung erhöht.

Dabei ist es für die Messung nicht von Bedeutung, ob die Elektroden mit dem relevanten Material nur beschichtet sind oder komplett daraus bestehen. Bei einer zu dünnen Beschichtung ergeben sich jedoch evtl. Probleme daraus, dass die Beschichtung bei einer Reinigung abgetragen werden kann. Daher besteht bevorzugt nicht nur die Oberfläche der Elektroden aus dem relevanten Material, sondern die ganze Elektrode. Dies kann sich von Elektrode zu Elektrode in dem Array unterscheiden.

In einer bevorzugten Ausführungsform weisen sowohl die Arbeitselektroden als auch die Gegenelektrode eine planare, insbesondere planparallele Oberfläche auf, wobei sich bevorzugt die Oberflächen in einer Ebene befinden. Die Form der Oberfläche ist bevorzugt rund. Es können jedoch auch Elektroden verwendet werden, bei denen die Oberfläche eine andere Geometrie aufweist, z.B. eckig (etwa dreieckig oder quadratisch) und/oder gewölbt.

Bevorzugt liegt der Durchmesser der Arbeitselektroden im Bereich zwischen 0,5 und 3 mm, z.B. zwischen ca. 0,8 mm und 2 mm, bevorzugt ca. 0,95-1 mm. Die Arbeitselektroden können sich in ihrer Geometrie und/oder ihrem Durchmesser unterscheiden. Bevorzugt weisen alle Arbeitselektroden die gleiche Geometrie ihrer Oberfläche und den gleichen Durchmesser auf.

Bevorzugt ist nur eine Gegenelektrode vorgesehen, die in der Mitte der Arbeitselektroden angeordnet ist. Es wurde gefunden, dass die Messung verbessert wird, wenn der Durchmesser der Gegenelektrode größer als der Durchmesser der Arbeitselektroden ist, z.B. ca. vierfach bis siebenfach so groß. Der Durchmesser der Gegenelektrode kann z.B. zwischen 4 und 8 mm liegen, bevorzugt ca. 6-7 mm.

Die Gegenelektrode kann bevorzugt aus einem metallischen oder nicht eisenmetallischen Werkstoff gefertigt oder als Beschichtung auf einem Trägermaterial aufgebracht sein, bevorzugt aus einem trinkwasserzugelassenen rostbeständigen Stahl. Weitere mögliche Materialien sind Gold oder Platin.

Auch ein Aufbau des Elektrodenarrays aus Nano- und/oder Mikroelektroden ist möglich. Bevorzugt weisen die Elektroden jedoch eine größere Oberfläche im Millimeter oder Zentimeterbereich auf. Dies ermöglicht etwa einen längeren Betrieb der Vorrichtung ohne Wartung und eine einfachere Reinigung.

In dem Elekrodenarray sind die Arbeitselektroden bevorzugt konzentrisch angeordnet, also symmetrisch um einen gemeinsamen Mittelpunkt. Bevorzugt sind die Arbeitselektroden kreisförmig um einen gemeinsamen Mittelpunkt angeordnet, in welchem sich die Gegenelektrode befindet.

In einer Ausführungsform kann der Abstand des Mittelpunkts der konzentrisch angeordneten Arbeitselektroden, z.B. jeweils mit einem Durchmesser von 0,95 mm, von dem Kreismittelpunkt, in dem sich die Gegenelektrode mit einem Durchmesser von ca. 6 mm befindet, ca. das 1,2-2fache des Durchmessers der Gegenelektrode betragen, z.B. ca. 9,3 mm.

Eine planare Geometrie von Arbeitselektroden und Gegenelektrode, mit der diese in Kontakt mit einer bei der Messung in der Messkammer enthaltenen Flüssigkeit treten können, ergibt sich bevorzugt daraus, dass andere Oberflächen dieser Elektroden nicht in Kontakt mit der Flüssigkeit treten können. Auf einer Seite sind diese Elektroden funktionell mit dem Impedanzanalysator verbunden. Ansonsten sind sie von der Flüssigkeit separiert, bevorzugt durch einen Isolator. Es wurde gefunden, dass Fähigkeiten des Isolators zur Isolation gegen Kontakt mit der Flüssigkeit und als elektrischer Isolator, sowie bevorzugt als Wärmeisolator wichtig sind. Der Isolator umfasst bevorzugt eine Glaskeramik, insbesondere Glaskeramik auf Basis einer Borosilikatglas-Matrix, oder besteht daraus. Borosilikatglas mit Glimmer, insbesondere Macor ® (Corning Inc., Corning, NY, USA) hat sich im Rahmen der Erfindung als besonders geeignet erwiesen. Macor ® setzt sich aus folgenden Bestandteilen zusammen: 6 % Siliciumdioxid (SiO2); 17 % Magnesium (MgO); 16 % Aluminiumoxid (Al2O3); 10 % Kalium (K2O); 7 % Bor (B2O3); 4 % Fluor (F). Macor ® ist ein sehr guter elektrischer Isolator (ρ > 10¹⁷ Ω cm) und bietet eine sehr gute Formstabilität und geringe Fertigungstoleranzen. Durch die Eigenschaften von Macor ® als effizienter Wärme- und Stromisolator und durch die gute Verarbeitbarkeit wird gewährleistet, dass die Elektroden voneinander elektrisch isoliert sind, im Sensorkopf fixiert sind und die elektrischen Anschlüsse zuverlässig gegen die zu analysierende Flüssigkeit abgedichtet sind. Alternativ können z.B. auch VITRONIT ® (Vitron, Jena-Maua, DE) oder Polyetheretherketon (abgekürzt PEEK), letzterer hat in Experimenten ebenfalls sehr gute Ergebnisse gezeigt, als Isolationsmaterial verwendet werden, die ähnliche Eigenschaften aufweisen.

Im Rahmen der Erfindung steht der unbestimmte Artikel"ein" oder "eine", sofern dies nicht explizit anders erwähnt ist, für "ein oder mehrere". In einer Ausführungsform der erfindungsgemäßen Vorrichtung umfasst das Elektrodenarray nur genau eine Arbeitselektode aus jedem der unterschiedlichen Materialien.

In einer bevorzugten Ausführungsform umfasst das Elektrodenarray mindestens jeweils zwei Elektroden des gleichen Materials, wobei auch hier bevorzugt die Elektroden konzentrisch und wie oben beschrieben angeordnet sind. In diesem Fall bedeutet dies, dass die Elektroden symmetrisch um einen gemeinsamen Mittelpunkt angeordnet sind und dass sich die Elektroden gleichen Materials punktsymmetrisch gegenüberliegen. Wenn mindestens zwei Elektroden des gleichen Materials vorgesehen sind, können Kontrollmessungen durchgeführt werden, durch die Störfaktoren, wie z.B. Blasenbildung oder Verschmutzungen an der Elektrodenoberfläche, detektiert und ggf. durch geeignete Maßnahmen, wie z.B. Reinigung der Elektrodenoberfläche, beseitigt werden können. Auch können fehlerhafte Messwerte, die sich durch solche Störfaktoren ergeben, durch Abweichungen zwischen den beiden Elektroden gleichen Materials erkannt, und - im Wesentlichen basierend auf Erfahrungswerten - ausgeschlossen werden.

Optional ist es möglich, die Messungen mehrerer, insbesondere zweier gleicher Elektroden, sofern die Messwerte nicht auf Störfaktoren hindeuten, bei der Auswertung zu mitteln oder getrennt in die Analyse mit einzubeziehen.

Die Elektroden des Elektrodenarrays sind in der Messkammer so angeordnet, und die Messkammer ist so ausgestaltet, dass bei einer Messung alle Elektroden in Kontakt mit in der Messkammer befindlicher Flüssigkeit sind. In einer Ausführungsform, insbesondere bei Verwendung der Vorrichtung, umfasst die Messkammer eine zu analysierende Flüssigkeit. Dabei ist die Flüssigkeit mit den Elektroden des Elektrodenarrays in Kontakt.

Die Messkammer umfasst auch mindestens einen Einlauf und Auslauf für die Flüssigkeit, wobei das Einlaufen und Auslaufen z.B. über Ventile gesteuert werden kann. Einlauf und Auslauf können als eine Öffnung oder zwei Öffnungen ausgestaltet sein. Bevorzugt handelt es sich um eine Durchflusskammer. Die Messkammer wird bevorzugt so ausgeformt, dass Toträume in der Fluidik vermieden werden. Optimal ist eine ellipsoid-förmige Ausformung der Messkammer mit dem Ziel eines verbesserten Strömungsverhaltens.

Die Analyse der zellulären Bestandteile in einer Flüssigkeit erfolgt erfindungsgemäß über elektrochemische Impedanzspektroskopie über einen bestimmten Frequenzbereich der Wechselspannung. Bevorzugt ist eine Messung in einem Frequenzbereich von 50 bis 10 000 Hz möglich, mehr bevorzugt einem Frequenzbereich von 10-100 000 Hz. Der in der Vorrichtung enthaltene Impedanzanalysator umfasst daher insbesondere ein Impedanzspektrometer, und ist dazu geeignet, die durch Messung mit dem Impedanzspektrometer erhaltenen Daten zu analysieren und/oder auszugeben. Die Elektroden sind bevorzugt so funktionell mit dem Impedanzspektrometer verbunden, dass eine separate Impedanzmessung aller Arbeitselektroden über den gewünschten Frequenzbereich nacheinander oder gleichzeitig möglich ist.

Die Vorrichtung kann ferner einen zur Analyse der Daten geeigneten Computer umfassen, der die ausgegebenen Daten verarbeitet. Alternativ - und bevorzugt - kann eine Vorrichtung zur Übermittlung der ausgegebenen, optional schon z.T. weiterverarbeiteten Daten an einen Computer oder ein Computernetzwerk vorgesehen sein. So können Daten z.B. übers Internet an ein Rechenzentrum weitergegeben werden, wo eine Analyse stattfinden kann. Auch eine Vorrichtung zum Empfang von Daten, etwa zur Steuerung der Vorrichtung, kann vorgesehen sein.

Bei der elektrochemischen Impedanzspektroskopie wird die Impedanz elektrochemischer Systeme als Funktion der Wechselspannung bzw. des Wechselstroms bestimmt. Wird eine Wechselspannung angelegt, bildet sich eine dielektrische Doppelschicht mit Ionen und/oder zellulären Bestandteilen der zu analysierenden Flüssigkeit an der Oberfläche der Arbeitselektrode aus, welche von der Ionenstärke und den Ionenarten der zu analysierenden Probe und den Oberflächeneigenschaften der Arbeitselektrode abhängen.

Es sind auch weitere elektrochemische Analysemethoden, wie z.B. galvanische, potentiometrische oder eine Kombination der Methoden, möglich, so dass die Vorrichtung entsprechend ausgestaltet sein kann. Auch eine Ausgestaltung der Vorrichtung, die sie für weitere Analysemethoden geeignet macht, ist möglich, z.B. für Temperaturmessung, Leitfähigkeitsmessung, pH-Messung, ein antikörperbasiertes Assay oder andere.

Im Rahmen der Erfindung umfasst die Vorrichtung bevorzugt einen Filter, welcher zum Entfernen von zellulären, insbesondere bakteriellen Bestandteilen aus der Flüssigkeit geeignet ist. Dieser kann z.B. eine Porengröße von maximal 0,22 µm, bevorzugt max. 0,1 µm oder von max. 0,02 µm aufweisen. In einer bevorzugten Ausführungsform ist der Filter ein selbstreinigender Bernoulli Filter. Dadurch wird ein Abtrennen der zellulären Bestandteile bei langen Filterstandzeiten ohne Verstopfung gewährleistet.

Durch eine gestaffelte Filterung mit verschiedenen Porengrößen lassen sich optional Untersuchungen auf Zellen bzw. Bakterien verschiedener Größen durchführen. Daher umfasst die Vorrichtung in einer Ausführungsform mehrere Filter verschiedener Porengrößen, die alternativ anzusteuern sind.

Bevorzugt umfasst die Vorrichtung ein Leitsystem, welches es ermöglicht, die Flüssigkeit aus der Messkammer durch den oder die Filter wieder in die Messkammer zu leiten. Mittels des Filters können dabei zelluläre Bestandteile der Flüssigkeit, z.B. bakterielle Zellen, zuverlässig aus der Flüssigkeit entfernt werden, wodurch eine Differenzmessung mit und ohne Zellen ermöglicht wird.

Optional umfasst die Vorrichtung Mittel zur Lyse von Zellen, wobei eine solche Lyse z.B. durch Erhitzen mittels einer Heizung, Bestrahlung mittels einer Strahlungsquelle (z.B. UV, radioaktiv, etwa gamma-Strahlung), Anwendung eines pulsierenden elektrischen Feldes (z.B. Hochspannungsimpulse) und/oder Ultraschall oder chemische Mittel, bevorzugt aber durch physikalische Mittel, erreicht werden kann. In einer Ausführungsform ist die Lyse in der Messkammer möglich, was optional gleichzeitig zur Reinigung der Messkammer dienen kann, alternativ ermöglicht ein Leitsystem, dass Flüssigkeit aus der Messkammer in eine Lyse-Kammer geleitet wird und nach einer Lyse wieder in die Messkammer geleitet wird.

Optional umfasst die Vorrichtung auch Mittel zur Kultivierung von Zellen und/oder Speicherung einer Probe, z.B. unter Kühlung (-18°C bis 4°C) oder unter zur Kultivierung geeigneten Bedingungen (z.B. 15-37°C), wobei eine Wiedereinleitung in die Messkammer nach Kultivierung oder eine manuelle Probenentnahme möglich sein kann.

Die Vorrichtung umfasst ferner bevorzugt ein Leitsystem, welches eine Einleitung von zu analysierender Flüssigkeit in die Messkammer erlaubt, wobei die Flüssigkeit bevorzugt aus einem Hauptstrom von Flüssigkeit abgeleitet werden kann, etwa aus einer Wasserleitung, z.B. Trinkwasserleitung. Auch Mittel zum Einleiten von Referenzlösung und/oder Spüllösung, z.B. Spülwasser können vorgesehen sein. Optional ist ein Speicher für Tenside, Lösungsmittel und/oder Biozide vorgesehen, welche der Spüllösung zugesetzt werden können.

Die Vorrichtung kann auch ein Leitsystem umfassen, welches Flüssigkeit aus der Messkammer wieder zu einem Hauptstrom der Flüssigkeit zuleitet, bevorzugt wird aber ein Abfallspeicher oder eine Einleitung in eine Abwasserleitung vorgesehen sein. Der Fluss der Flüssigkeiten in der Vorrichtung kann durch Ventile gesteuert werden, welche z.B. durch ein Computerprogrammprodukt gesteuert werden können.

Das Leitsystem ist bevorzugt ein fluidisches System.

Weiterhin sollte in der Vorrichtung mindestens ein Mittel vorgesehen sein, welches eine Dekontamination der Messkammer und bevorzugt des Filters und des Leitsystems, erlaubt, also eine Minimierung von residualen zellulären Bestandteilen und/oder des Biofilms. Zur Dekontamination ist z.B. Spüllösung, welche z.B. ein organisches Lösungsmittel wie Ethanol oder Propanol oder eine Mischung daraus umfassen kann oder eine Tensidlösung geeignet. Bevorzugt wird mit deionisiertem Wasser oder auch einer verdünnten Zitronensäure gespült und/oder eine elektrochemisch aktivierte Desinfektionslösung eingesetzt, da diese in niedrigen Konzentrationen in den Abfluss geleitet werden können. Optional kann eine Heizung der Spüllösung und/oder Heizung der Messkammer die Dekontamination unterstützen. Eine Erweiterung mit einer Luft-Impuls-Spülung kann den Reinigungseffekt durch Turbulenzen in Teilen oder dem gesamten Sensor-System verstärken, was jedoch den Nachteil einer Blasenbildung zur Folge haben kann. In diesem Fall sind also Mechanismen zur Kontrolle von Blasenbildung (z.B. je zwei gleiche Arbeitselektroden) besonders sinnvoll. Auch Mittel zur Bestrahlung (z.B. UV-Bestrahlung oder radioaktive Bestrahlung) und Mittel zur Ultraschallbehandlung können zur Dekontamination dienen. Bevorzugt werden mehrere Mittel kombiniert, z.B. Spüllösung, Heizung der Spüllösung und UV-Bestrahlung.

Vorteilhafterweise ist mit einer erfindungsgemäßen Vorrichtung eine online-Überwachung von zellulären Bestandteilen einer Flüssigkeit möglich, bevorzugt eine online-Überwachung z.B. von Trinkwasser auf bakterielle zelluläre Bestandteile. Unter online-Überwachung wird im Rahmen der Erfindung ein im Wesentlichen, bevorzugt, vollständig automatisches Überwachungs-Verfahren verstanden, welches im Wesentlichen oder vollständig ohne manuelles Eingreifen ablaufen kann und eine Mess- und Auswertezeit von einer Stunde oder weniger, bevorzugt von 30 Minuten oder weniger, für eine Messprobe benötigt. Dies wird als im Wesentlichen kontinuierliche Analyse beschrieben.

Die Erfindung betrifft weiterhin ein Verfahren zur quantitativen und/oder qualitativen Analyse von zellulären Bestandteilen einer Flüssigkeit, wobei das Verfahren Schritte umfasst, bei denen man
a) die Flüssigkeit in die Messkammer der erfindungsgemäßen Vorrichtung einleitet;
b) eine Impedanzmessung durchführt;
c) eine Analyse der in Schritt b) erhaltenen Impedanzwerte, bevorzugt Impedanzspektren durchführt.

Erfindungsgemäß wird bevorzugt eine quantitative, besonders bevorzugt zusätzlich eine qualitative Analyse zellulärer Bestandteile einer Flüssigkeit durchgeführt. Die qualitative Analyse erlaubt insbesondere Aussagen über Größe und/oder Art der zellulären Bestandteile.

Zur Analyse wird eine elektrochemische Impedanzspektroskopie durchgeführt, die bevorzugt mit einer konstanten Spannungsamplitude oder in einer anderen Ausführung mit einem konstantem Strom betrieben wird.

Die Einleitung der Flüssigkeit in Schritt a) kann ohne Filtrierung oder nach einer Filtrierung zur Entfernung zellulärer Bestandteile z.B. über einen hierin beschriebenen Filter, und damit zum Erstellen einer Nullprobe, erfolgen. Zu Beginn jedes Messzyklus wird typischerweise eine Nullprobe 1, bevorzugt eine zuvor filtrierte Probe ohne zelluläre Bestandteile, hergestellt und eine Impedanzanalyse durchgeführt. Die erhaltenen Impedanzspektren dienen bevorzugt als Referenz- oder Vergleichswerte für folgende Messungen. Anschließend wird eine Impedanzanalyse einer Testprobe der zu analysierenden Flüssigkeit durchgeführt und die Werte mit den Werten der Nullprobe verglichen. Bevorzugt umfasst das erfindungsgemäße Verfahren eine im Wesentlichen kontinuierliche online-Messung von Testproben, wobei bevorzugt mindestens alle 24 h, mindestens alle 12 Stunden, mindestens alle 6 Stunden, mindestens alle 4 Stunden, mindestens alle 2 Stunden, mindestens alle Stunde oder mindestens alle halbe Stunde eine Testprobe analysiert wird.

Die Impedanzmessung in Schritt b) erfolgt bevorzugt durch Messung der Impedanz über einen bestimmten Frequenzbereich der Wechselspannung. Die Impedanz wird bevorzugt an jeder der Arbeitselektroden nacheinander über den gleichen Frequenzbereich der angelegten Wechselspannung bestimmt. Bevorzugt wird jede der Arbeitselektroden die Impedanz für einen Frequenzbereich von 10 bis 100 000 Hz, bevorzugt für einen Frequenzbereich von 50 bis 10 000 Hz gemessen. Die Erfinder haben festgestellt, dass es vorteilhaft ist, die Impedanz bei mindestens 192 verschiedenen Frequenzen zu messen, wodurch sich für jede der Arbeitselektroden mindestens 192 Stützstellen ergeben. Diese dienen zur Interpolation der Funktion der Impedanz in Abhängigkeit von der Frequenz der Wechselspannung.

Bevorzugt umfasst Schritt c) eine multivariate Datenverarbeitung, welche
i) die Impedanzwerte übernimmt;
ii) optional die Impedanzwerte filtert, um Ausreißer auszusortieren und/oder Durchschnittswerte mehrerer Messungen bildet;
iii) die erste und zweite Ableitung der Impedanzspektren oder eines Bode-Diagramms, bevorzugt als Funktion des Betrages der Impedanzen, bildet; und
iv) die erhaltenen Ergebnisse mit Referenzwerten vergleicht;
wobei als Ergebnis der Datenverarbeitung eine quantitative und/oder qualitative Aussage über zelluläre Bestandteile der Flüssigkeit ausgegeben wird. Bevorzugt erfolgt die Ausgabe als Anzeige einer Auswahl aus vorher definierten quantitativen Aussagen über die Menge zellulärer Bestandteile in der Flüssigkeit, am meisten bevorzugt als ampelartige Anzeige. Die Datenverarbeitung und/oder Ausgabe müssen nicht vor Ort erfolgen, sondern die Impedanzwerte, Zwischenergebnisse der Datenverarbeitung oder das Ergebnis der Datenverarbeitung kann auch über eine Datenverbindung weitergeleitet werden, z.B. an ein Rechenzentrum, eine Analysestelle und/oder einen menschlichen Entscheider, z.B. in einem Wasserwerk oder einer Kontrollstelle für Wasserqualität, insbesondere bei Trinkwasser.

Die Erstellung des Bode-Diagramms in Schritt iii) kann für den Betrag (Amplitudengang) und/oder für das Argument (Phasengang) der komplexwertigen Funktion der Impedanz erstellt werden.

Um eine Unabhängigkeit der Charakteristika verschiedener Wassersorten zu erreichen, wird jeweils die prozentuale Änderung zwischen dem Nullwert und dem Probenwert normalisiert nach dem Nullwert als Koeffizient der Variation berechnet.

Bevorzugt erfolgt die Analyse der Impedanz in Abhängigkeit der Frequenz der angelegten Wechselspannung. Es ist möglich, über rechnerische Mittel, insbesondere eine FourierTransformation, auch einen Abhängigkeit von Zeit (Time-Domain) zu analysieren.

Bevorzugt werden als Referenzwerte in Schritt iv) Ergebnisse, welche mit gleichem Verfahren für eine wie hierin beschrieben filtrierte Nullprobe 1 erhalten wurden, welche also keine zellulären Bestandteil umfasst, verglichen. Alternativ kann auch eine Nullprobe 1 verwendet werden, welche zelluläre Bestandteile umfasst, wobei diese in einer Konzentration vorliegen, die in der Testprobe erwünscht oder tolerierbar ist.

Der Vergleich in Schritt iv) kann den Vergleich eines Bode-Diagramms als Funktion des Betrags oder der Phase der Impedanzspektren, optional beider Bode-Diagramme umfassen. Die Erfinder haben überraschenderweise festgestellt, dass es die Aussagekraft der Ergebnisse nicht erhöht und daher nicht notwendig ist, beide Bode-Diagramme der Impedanzspektren auszuwerten. Es wird bevorzugt das Bodediagramm als Funktion des Betrages der Impedanzen analysiert. Der Vergleich in Schritt iv) umfasst in einer Ausführungsform einen Vergleich der ersten und zweiten Ableitung der Impedanzspektren bzw des Bodediagrammes mit jeweiligen Referenzwerten.

Durch den Vergleich der Ableitungen ist es möglich, invariante Charakteristika der Flüssigkeit ohne zelluläre Verunreinigungen zu eliminieren. Um eine Unabhängigkeit von den Charakteristika verschiedener Flüssigkeiten, insbesondere, verschiedener Wassersorten, zu erreichen wird bevorzugt jeweils die prozentuale Änderung zwischen dem Referenzwert und dem Testwert normalisiert nach dem Nullwert als Koeffizient der Variation berechnet.

Der Vergleich in Schritt iv) umfasst optional das Ableiten spezifischer Muster, welche quantitative und/oder qualitative Daten umfassen können, aus den vorverarbeiteten Daten, welche gewichtet werden können; wobei auf Basis dieser Muster ein maschinelles Lernverfahren angewendet werden kann, welches die entscheidenden Muster extrahiert und mit Referenzmustern, z.B. für Wasser ohne zelluläre Bestandteile, vergleicht.

Bevorzugt werden sich regelmäßig im Tagesverlauf ändernde Impedanzwerte, z.B. bei Änderung der Temperatur der Flüssigkeit, in die multivariate Datenanalyse einbezogen.

In einer bevorzugten Ausführungsform erfolgt die multivariate Datenauswertung, insbesondere der Vergleich mit Referenzwerten, mit Hilfe eines künstlichen neuronalen Netzwerkes.

Bei signifikanten Abweichungen der erhaltenen Impedanzwerte und/oder, bevorzugt, der daraus durch Datenverarbeitung erhaltenen Ergebnisse zu den Referenzwerten, insbesondere den Referenzwerten der Nullprobe 1, wird bevorzugt die zu analysierende Flüssigkeit von zellulären Bestandteilen befreit, insbesondere, indem sie durch einen hierin beschriebenen Filter geleitet wird, um eine Nullprobe 2 zu erhalten. Dann wird das das erfindungsgemäße Verfahren für die Nullprobe 2 durchgeführt, wobei die Abweichungen der Werte der Testprobe von den Werten der Nullprobe 1 auf zelluläre Bestandteile hinweisen, wenn Nullprobe 1 und 2 im vergleichbare, bevorzugt im Wesentlichen identische Daten aufweisen. Unter signifikanter Abweichung werden vorher festgelegte kritische Bereiche verstanden, die auf Erfahrungswerten oder Eichwerten beruhen, z.B. Ergebnissen mit bekanntermaßen zelluläre Bestandteile umfassenden Flüssigkeiten. Die zu analysierende Flüssigkeit ist, um einen möglichst guten Vergleich zu ermöglichen, bevorzugt zeitnah nach der Entnahme der ersten Probe entnommene Vergleichsflüssigkeit. Alternativ ist es möglich, vor einer Probenmessung ein größeres Volumen Probe als benötigt zwischenzuspeichern und einen für die erste Messung nicht benötigten Teil davon zu filtrieren und als Nullprobe 2 zu verwenden.

Bevorzugt wird die Messkammer nach Detektion einer potentiell zelluläre Bestandteile enthaltenen Probe gereinigt, z.B. gespült und/oder aufgeheizt.

In einer Ausführungsform wird bei dem erfindungsgemäßen Verfahren mindestens eine weitere Maßnahme durchführt, ausgewählt aus der Gruppe umfassend
a) Lyse zellulärer Bestandteile und Bestimmung von Impedanzwerten vor und nach Lyse. Dies kann zur Verstärkung von Meßsignalen und Erhöhung der Sensititvität führen, wobei die Lyse z.B. durch Anwendung eines pulsierenden elektrischen Feldes (z.B. Hochspannungsimpulse), Bestrahlung, Hitze und/oder Ultraschall, durchgeführt wird und optional nach einer Kultivierung von Zellen stattfinden kann; die Vorrichtung umfasst bevorzugt geeignete Mittel zur Durchführung der Zelllyse, wobei die Lyse optional bei jeder Messung und/oder nur bei einer signifikanten Abweichung von den Referenzwerten erfolgen kann;
b) eine weitere elektrochemische, biochemische oder physikalische Messung, ausgewählt aus der Gruppe umfassend Temperaturmessung, Leitfähigkeitsmessung, pH-Messung, und antikörperbasiertes oder aptamerebasierte Assay. Vorteilhafterweise kann mit einem antikörperbasierten oder aptamerebasierten Assay die Klasse, Gattung und/oder Spezies der Bakterien bestimmt werden. Antikörperbasierte Messungen, welche automatisiert ablaufen können, können z.B. ELISA, EIA, Immunfluoreszenzmessung, durchflusszytometrische Messungen oder ähnliches sein.
c) Speicherung und/oder Veranlassung der Entnahme einer Probe der Testprobe der Flüssigkeit bei Feststellung von zellulären Bestandteilen in der Testprobe. Optional wird eine automatische Entnahme und Speicherung einer Probe, bei der die erhaltenen Ergebnisse auf eine möglicherweise kritische Verunreinigung mit zellulären Bestandteilen hinweisen (bevorzugt unter Kühlung) und/oder ein Versenden der Probe an ein Labor zur weiteren Untersuchung veranlasst.

Die erfindungsgemäße Detektionsvorrichtung erkennt vorteilhafterweise mikrobiologische Belastungen z.B. im Trinkwasser einer Trinkwasserinstallation erlaubt eine quantitative Bewertung z.B. in Form eines etwa ampelartigen Warnsignales. Eine geringe Anzahl von Bakterien bzw. Zellen kann von einer erhöhten bzw. einer sehr hohen Zellanzahl unterschieden werden. Alle im Trinkwasser enthaltenen mikrobiellen Kontaminationen werden erfasst und in die Differenzierung einbezogen. Einflüsse von anorganischen Stoffen, Temperatur und weiteren Störgrößen einer Trinkwasserinstallation können von einer mikrobiellen Belastung unterschieden werden. Zusätzlich ist es vorteilhafterweise möglich, auch qualitative Unterschiede zwischen enthaltenen zellulären Bestandteilen zu differenzieren, etwa anhand der Größe der Bakterien. So können z.B. Hinweise auf pathogene, meist kleinere Bakterien, von Hinweisen auf im allgemeinen nicht-pathogene größere Bakterien differenziert werden, und optional bei Bedarf nötige Zusatzuntersuchungen (z.B. b, c) zur Differentialanalyse eingeleitet werden.

Erfindungsgemäß können z.B. Konzentrationen von Bakterien ab 100 Zellen/ml bevorzugt ab 1000 Zellen / ml nachgewiesen werden. Dafür ist eine vorgängige Erhöhung der Konzentration der Zellen bzw. Bakterien z.B. über eine Kultivierung, Filtration oder Zentrifugation nicht nötig.

In einer bevorzugten Ausführungsform umfasst die erfindungsgemäße Vorrichtung Mittel zur Steuerung und Auswertung des erfindungsgemäßen Verfahrens, wobei die Mittel bevorzugt einen Computer mit einem Computerprogrammprodukt zur Steuerung und/oder Auswertung des erfindungsgemäßen Verfahrens umfassen. Der Computer umfasst bevorzugt Speichermittel, Eingabemittel und Ausgabemittel. Dabei können Eingabemittel und Ausgabemittel auch Mittel sein, die eine Datenübertragung an einen an anderem Orte befindlichen Analysecomputer, Computersystem oder Kontroll- und Rechenzentrum erlauben. Die Steuerung der erfindungsgemäßen Vorrichtung umfasst mindestens eine, bevorzugt mehrere oder alle der folgenden Aufgaben: die Steuerung von Einlauf und Auslauf der Flüssigkeit in die Messkammer, die Ansteuerung der verschiedenen Arbeitselektroden zur aufeinanderfolgenden Messung, die Filtration der gemessenen Flüssigkeiten bei signifikanten Unterschieden zur Nullprobe 1 und Rückführung in die Messkammer, und die Steuerung von Reinigungsvorgängen und optional Lysevorgängen.

In einer Ausführungsform betrifft die Erfindung ein System, umfassend einen Computer, auf dem das Computerprogrammprodukt ausgeführt wird, und welches bevorzugt auch die Messvorrichtung der Erfindung umfasst, welche funktionell mit einem Leitsystem für Flüssigkeiten, z.B. einer Trinkwasserleitung, verbunden ist.

Die Durchführung der erfindungsgemäßen Analyse, welche auch auf einem örtlich ausgegliederten Computer oder Computersystem durchgeführt werden kann, wird bevorzugt zumindest teilweise mittels eines künstlichen neuronalen Netzwerkes durchgeführt. Dieses kann ein durch Software simuliertes künstliches neuronales Netzwerk oder ein Hardware-implementiertes künstliches neuronales Netzwerk sein, es kann aber auch ein weiteres geeignetes numerisches multivariates Diskriminierungs- bzw. Regressionsverfahren, wie zum Beispiel support vector machines oder andere eingesetzt werden.

Der Anwendung des erfindungsgemäßen Verfahrens im Testbetrieb kann eine Anwendung im Trainingsbetrieb vorangehen, bei der die Analyseverfahren, insbesondere das künstliche neuronale Netzwerk anhand von Eichproben bekannter Bakterienkonzentration und Bakterienart trainiert werden.

Die Erfindung stellt auch ein Computerprogrammprodukt bereit, welches in den internen Speicher eines Computers geladen werden kann oder auf einem computerlesbaren Speichermedium gespeichert ist. Dieses umfasst computerlesbare Programm-Mittel zur Steuerung der erfindungsgemäßen Vorrichtung bei der Durchführung des erfindungsgemäßen Verfahrens oder bei der Durchführung des Analyseschrittes des erfindungsgemäßen Verfahrens, wenn das Computerprogrammprodukt auf einem Computer ausgeführt wird. Dabei umfasst die Analyse bevorzugt die Simulation eines neuronalen Netzwerkes.

Dabei können verschiedene Computerprogrammprodukte vorgesehen sein, die miteinander kommunizieren können. So kann die erfindungsgemäße Messvorrichtung von einem Computerprogrammprodukt gesteuert werden, welches geeignete Programm-Mittel umfasst und ferner einen Transfer der erhaltenen Messdaten an einen entfernt gelegenen Computer oder Computersystem übermitteln kann, wo die Auswertung der Daten stattfinden kann. Diese wird dann von einem zweiten Computerprogrammprodukt durchgeführt, welches bevorzugt die Durchführung der erfindungsgemäßen Analyse zumindest teilweise mittels eines künstlichen neuronalen Netzwerkes durchführt. Das zweite Computerprogrammprodukt umfasst jedoch auch Mittel zur Steuerung der Vorrichtung, da einige Verfahrensschritte (z.B. Filtration der Flüssigkeit und/oder Reinigung von Elektroden, der Messkammer oder der gesamten Vorrichtung) nur oder bevorzugt bei bestimmten Ergebnissen, z.B. Ergebnissen der Analyse, die auf ein Vorhandensein zellulärer Bestandteile oder eine Verunreinigung einer oder mehrerer Elektroden hindeuten, durchgeführt werden sollen. Diese Steuerung kann ebenfalls über eine Datenübertragung vermittelt werden.

Die Erfindung betrifft auch die Verwendung der erfindungsgemäßen Vorrichtung, des erfindungsgemäßen Verfahrens oder des erfindungsgemäßen Computerprogrammprodukts zur quantitativen und/oder qualitativen Analyse von zellulären Bestandteilen einer Flüssigkeit, bevorzugt zur quantitativen und qualitativen Analyse von Trinkwasser auf bakterielle zelluläre Bestandteile.

### Kurze Beschreibung der Figuren

Bevorzugte weitere Ausführungsformen der Erfindung werden durch die nachfolgende Beschreibung der Figuren näher erläutert. Dabei zeigen:
- Fig. 1: eine schematische Darstellung des Sensorkopfes;
- Fig. 2.: ein Schnittbild des Sensorkopfes entlang der Schnittlinie A-A;
- Fig. 3: eine schematische Darstellung einer bevorzugten Vorrichtung (Handyscope HS5: Oszillator zur Auswertung; MUX: Multiplexer);
- Fig. 4: das Prinzip des Detektionssystem und
- Fig. 5: das Prinzip der multivariaten Datenverarbeitung
- Fig. 6: Bode Plots des Betrags der Impedanz für eine Arbeitselektrode, prozentuale Änderung zwischen dem Nullwert und dem Probenwert normalisiert nach dem Nullwert
A) Temperaturunterschiede zwischen 29 und 31 °C, Nullwert 29°C, unterste Kurve 29,5°C, zweite Kurve von unten 30°C, dritte Kurve von unten 30,5°C, oberste Kurve 31 °C.
B) Differenzen zwischen unterschiedlichen KCl-Lösungen 1mM und 5mM, Nullwert 1 mM
C) Differenzen zwischen Wasser mit und ohne Filtrierung, Nullwert: Filtriert
D) Differenzen zwischen Wasser mit 3,35 mM KCl (Nullwert) mit und ohne E. coli (untere Kurve 1x10⁵ /ml, obere Kurve 1x10⁷ /ml)
E) Differenzen zwischen Wasser mit 3,35 mM KCl (Nullwert) mit und ohne P. aeruginosa (untere Kurve 1x10⁵ /ml, obere Kurve 1x10⁷ /ml)

### Bezugszeichenliste

- 10: Messkammer
- 11: Elektrodenarray
- 13: Gegenelektrode
- 14: Elektroden/Arbeitselektroden
- 15: Oberfläche der Elektroden zur Flüssigkeit hin
- 16: Oberfläche der Elektroden zum Sensorkopf hin
- 17: Isolator
- 18: Sensorkopf
- 19: Markierungsbohrung für die erste Arbeitselektrode zur Vermeidung von Verwechslungen.
- 30: Filter
- 31: Leitsystem
- 32: Zuleitung
- 33: Auslauf
- 34: Pumpe
- 35: Abfallbehälter
- 36: Heizung
- 37: Behälter Heißspüllösung
- 38: Behälter Referenz

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

### 1. Eine bevorzugte Vorrichtung

Die Erfindung stellt eine Vorrichtung zur quantitativen und/oder qualitativen Analyse von zellulären Bestandteilen einer Flüssigkeit zur Verfügung, welche eine Messkammer **10** mit einem Elektrodenarray **11** mit einer Gegenelektrode **13** und einen Impedanzanalysator umfasst.

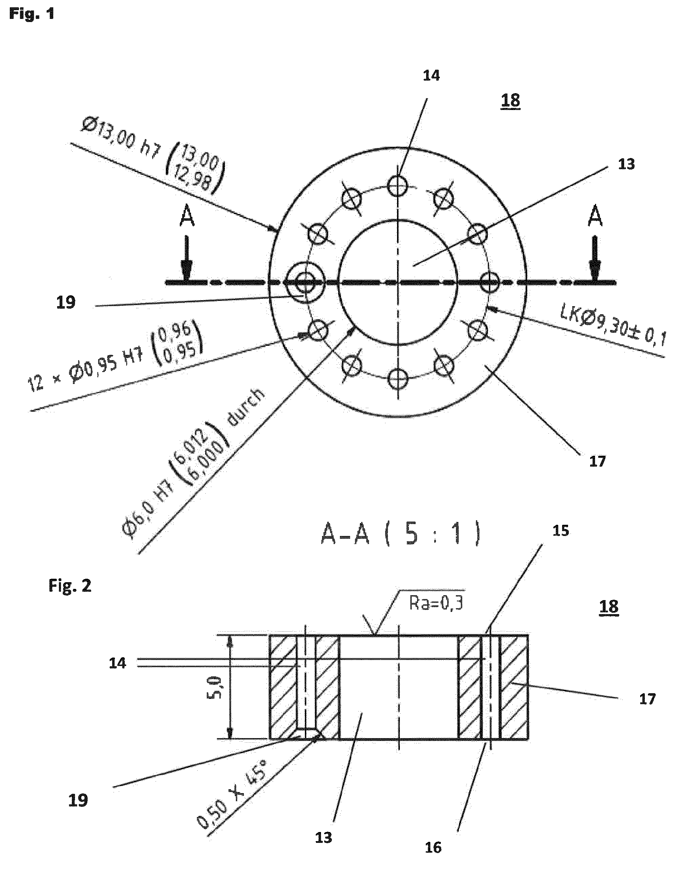

Figur 1 zeigt einen Sensorkopf **18,** welcher ein Elektrodenarray **11** aufweist. Das Elektrodenarray **11** umfasst zwölf Arbeitselektroden **14,** von denen jeweils zwei gleich sind und sich diese Paare in Bezug auf ihr Material unterscheiden. Die Arbeitselektroden **14** weisen eine Oberfläche aus Au, Ir, Pd, Pt, Rh und Re auf und bestehen bevorzugt komplett aus diesem Material.

Die Arbeitselektroden und die Gegenelektrode weisen eine planparallele runde Oberfläche (15, 16) auf. Der Durchmesser der Arbeitselektroden liegt bei 0,95-0,96 mm. Der Durchmesser der Gegenelektrode ist 6-6,012 mm. Die Gegenelektrode besteht aus rostbeständigem Stahl.

In dem Elekrodenarray **11** sind die Arbeitselektroden 14 konzentrisch angeordnet, also symmetrisch um einen gemeinsamen Mittelpunkt, in welchem sich die Gegenelektrode **13** befindet. Der Abstand des Mittelpunkts der konzentrisch angeordneten Arbeitselektroden **14** von dem Kreismittelpunkt, ist 9,3 +/-0,1 mm.

Figur 2 ist ein Schnittbild des Sensorkopfes **18** entlang der Schnittlinie A-A. Die Arbeitselektroden **14** und die Gegenelektrode **13** weisen eine planparallele, runde Oberfläche **15** auf, welche mit der zu analysierenden Flüssigkeit in Kontakt kommt und eine weitere planparallele runde Oberfläche **16,** über die der Kontakt zum Sensorkopf **18** hergestellt werden kann, so dass die Arbeitselektroden **14** funktionell mit dem Impedanzanalysator verbunden sind, an der gegenüberliegenden Seite können sie in Kontakt mit in der Messkammer **10** enthaltener Flüssigkeit treten. Ansonsten sind sie von einem Isolator **17** aus Macor ®. (Corning Inc., Corning, NY, USA).

Bevorzugt ist das Array **11** aus Arbeitselektroden **14** und Gegenelektrode **13** auf einem Sensorkopf **18** angeordnet, wobei der Sensorkopf **18** in eine leitfähige Fassung einschraubbar ist, welche, wenn die Messkammer bei der Messung eine Flüssigkeit umfasst, mit dieser in Kontakt tritt.

Die Elektroden **14** des Elektrodenarrays **11** sind in der Messkammer **10** so angeordnet, und die Messkammer **10** ist so ausgestaltet, dass bei einer Messung alle Elektroden **14** in Kontakt mit in der Messkammer **10** befindlicher Flüssigkeit sind. Bei Verwendung der Vorrichtung, umfasst die Messkammer **10** eine zu analysierende Flüssigkeit. Dabei ist die Flüssigkeit mit den Elektroden **14** des Elektrodenarrays **11** in Kontakt.

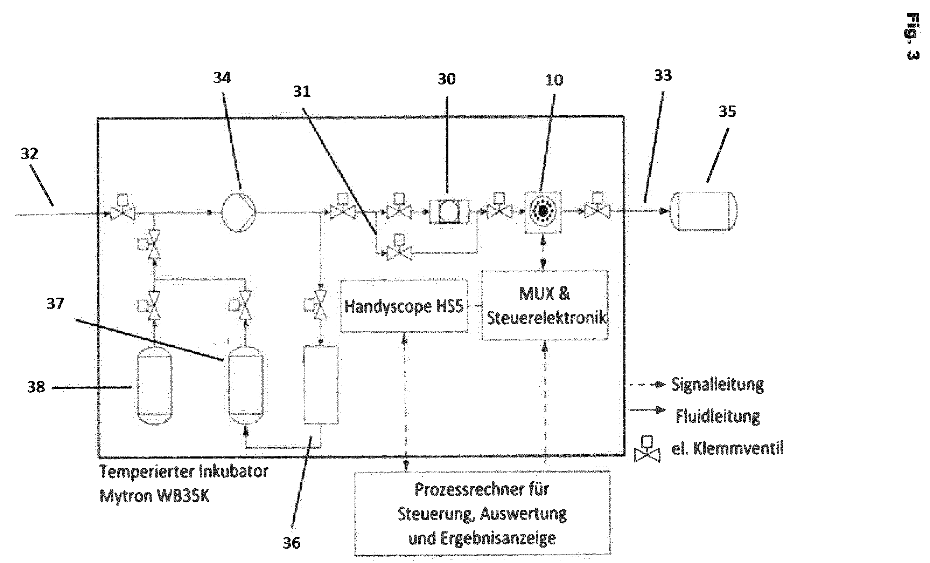

Figur 3 ist eine schematische Darstellung einer bevorzugten Vorrichtung (Handyscope HS5: Oszillator zur Auswertung; MUX: Multiplexer), wobei die Fluidströme und die Signalleitung dargestellt sind. Über eine Zuleitung **32** wird das Medium mittels einer Pumpe **34** über einen Filter und/oder ein Leitsystem **35** in die Messkammer **10** geleitet und kann über den Auslauf **33** in einen Abfallbehälter **36** geleitet werden. Die Messkammer **10** umfasst auch mindestens einen Einlauf und Auslauf für die Flüssigkeit, wobei das Einlaufen und Auslaufen z.B. über Ventile gesteuert werden kann. Einlauf und Auslauf können als eine Öffnung oder zwei Öffnungen ausgestaltet sein. Bevorzugt wird eine Durchflussmesskammer, welche einen gleichmäßigen Volumenstrom innerhalb der Messkammer **10** erlaubt.

Es ist ein Filter **30** vorgesehen, welcher zum Entfernen von bakteriellen Bestandteilen aus der Flüssigkeit geeignet ist und eine Maschenweite von 0,02 µm aufweist und als Bernoulli Filter ausgestaltet ist. Ein Leitsystem **31** ermöglicht, die Flüssigkeit aus der Messkammer **10** durch den Filter **30** wieder in die Messkammer **10** zu leiten.

Optional umfasst die Vorrichtung Mittel zur Lyse von Zellen, wobei eine solche Lyse z.B. durch Erhitzen mittels einer Heizung, Bestrahlung mittels einer Strahlungsquelle (z.B. UV, radioaktiv, etwa gamma-Strahlung), Anwendung eines pulsierenden elektrischen Feldes (z.B. Hochspannungsimpulse) und/oder Ultraschall oder chemische Mittel, bevorzugt aber durch physikalische Mittel, erreicht werden kann. In einer Ausführungsform ist die Lyse in der Messkammer möglich, was optional gleichzeitig zur Reinigung der Messkammer dienen kann, alternativ ermöglicht ein Leitsystem, dass Flüssigkeit aus der Messkammer in eine Lyse-Kammer geleitet wird und nach einer Lyse wieder in die Messkammer geleitet wird. In Figur 3 ist beispielhaft eine Heizung **36** dargestellt. Zudem sind ein Behälter zur Aufnahme der Heißspüllösung **37** und ein Behälter für die Referenzlösung **38** vorgesehen.

Optional umfasst die Vorrichtung auch Mittel zur Kultivierung von Zellen und/oder Speicherung einer Probe, z.B. unter Kühlung (-18°C bis 4°C) oder unter zur Kultivierung geeigneten Bedingungen (z.B. 15-37°C), wobei eine Wiedereinleitung in die Messkammer nach Kultivierung oder eine manuelle Probenentnahme möglich sein kann. Die Mittel zur Kultivierung von Zellen und/oder Speicherung einer Probe sind vorliegend nicht dargestellt.

Die Vorrichtung umfasst ferner ein fluidisches Leitsystem **31,** welches eine Einleitung von zu analysierender Flüssigkeit in die Messkammer **10** erlaubt, wobei die Flüssigkeit bevorzugt aus einem Hauptstrom von Flüssigkeit abgeleitet werden kann, insbesondere einer Trinkwasserleitung. Auch Mittel zum Einleiten von Referenzlösung und/oder Spüllösung, z.B. Spülwasser sind vorgesehen. Dabei ist ein Speicher für Tenside vorgesehen, welche der Spüllösung zugesetzt werden können.

Es ist eine Einleitung in eine Abwasserleitung für verwendete Meß- und Spülflüssigkeit vorgesehen. Der Fluss der Flüssigkeiten in der Vorrichtung kann durch Ventile gesteuert werden, welche durch ein Computerprogrammprodukt gesteuert werden können.

Weiterhin ist in der Vorrichtung mindestens ein Mittel vorgesehen, welches eine Dekontamination der Messkammer und des Filters **30** und des Leitsystems **31,** erlaubt. Zur Dekontamination ist Spüllösung, insbesondere eine Tensidlösung geeignet. Eine Heizung der Spüllösung und/oder Heizung der Messkammer kann die Dekontamination unterstützen. Ferner sind optional Mittel zur Bestrahlung (z.B. UV-Bestrahlung) und Mittel zur Ultraschallbehandlung vorgesehen.

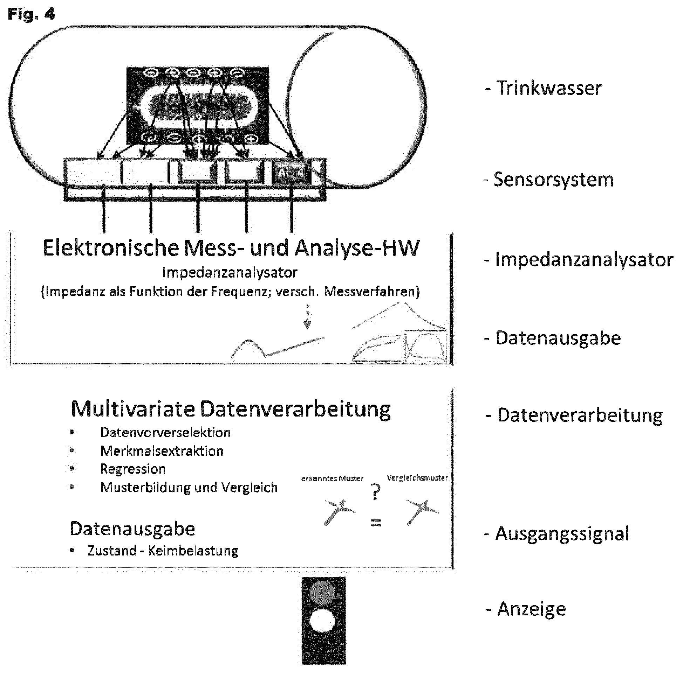

Figur 4 zeigt das Prinzip des Detektionssystems. Hierbei wird die zu analysierende Flüssigkeit (z.B. Trinkwasser) einem Sensorsystem zugeführt, welches eine Impedanzspektroskopie, d.h. eine Messung der Impedanz der Flüssigkeit über ein bestimmtes Impedanzspektrum, durchführt. Die gemessenen Werte werden mittels eines Impedanzanalysators analysiert und die gemessenen Daten an eine multivariate Datenverarbeitung ausgegeben. Die multivariate Datenverarbeitung umfasst eine Datenvorselektion, eine Merkmalsextraktion, eine Regression und eine abschließenden Musterbildung mit einem Vergleich der erkannten Muster mit Vergleichsmustern. Die Datenausgabe in Form eines Ausgangssignals wird über eine ampelartige Anzeige visualisiert, wodurch eine Aussage über den Zustand der Keimbelastung gemacht werden kann.

### 2. Datenverarbeitung

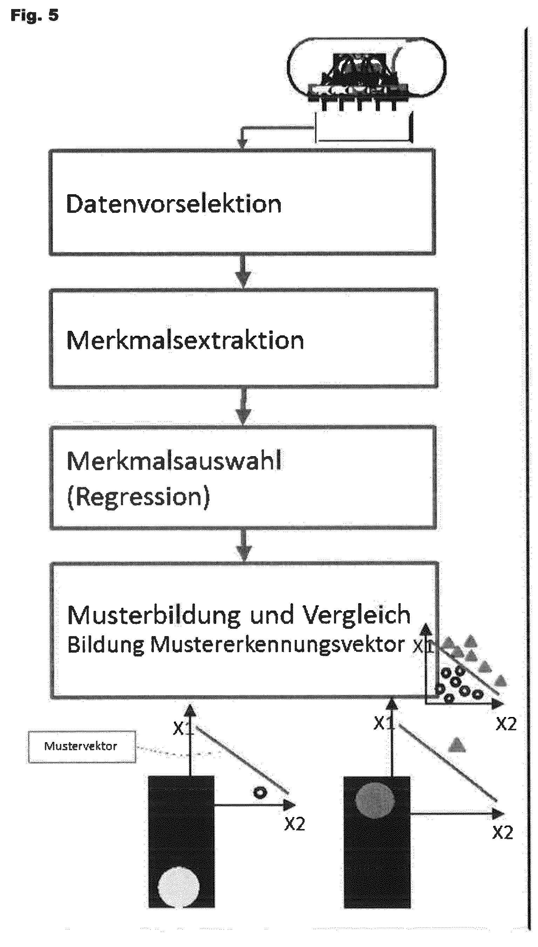

Figur 5 zeigt das Prinzip der multivariaten Datenverarbeitung, bestehend aus folgenden Schritten: Datenvorselektion, Merkmalsextraktion, Merkmalsauswahl (Regression), Musterbildung und Vergleich mit bekannten Mustern.

Am Anfang eines Messzyklus wird eine Nullprobe, welche bevorzugt durch Filtrierung über einen geeigneten Filter keine Bakterien oder anderen zellulären Bestandteile aufweist, in der unter 1. beschriebenen Vorrichtung untersucht. Nacheinander werden für die einzelnen Elektroden Impedanzspektren über einen Frequenzbereich von 10-100 000 Hz aufgenommen. Dabei werden mindestens 192 Stützstellen abgefragt.

Evtl. Ausreißer, die sich evtl. durch Gasblasen oder Verunreinigungen ergeben können, werden aussortiert. Es ist möglich, die Genauigkeit über Mehrfachmessungen der gleichen Elektroden oder von Elektroden des gleichen Materials und Bildung der Duchschnittswerte zu erhöhen.

Die gemessenen Impedanzwerte der einzelnen Elektroden werden als Bode-Plot, insbesondere als Bode-Plot des Betrags der Impedanz dargestellt. Die erste und zweite Ableitung der Bode-Plots wird gebildet.

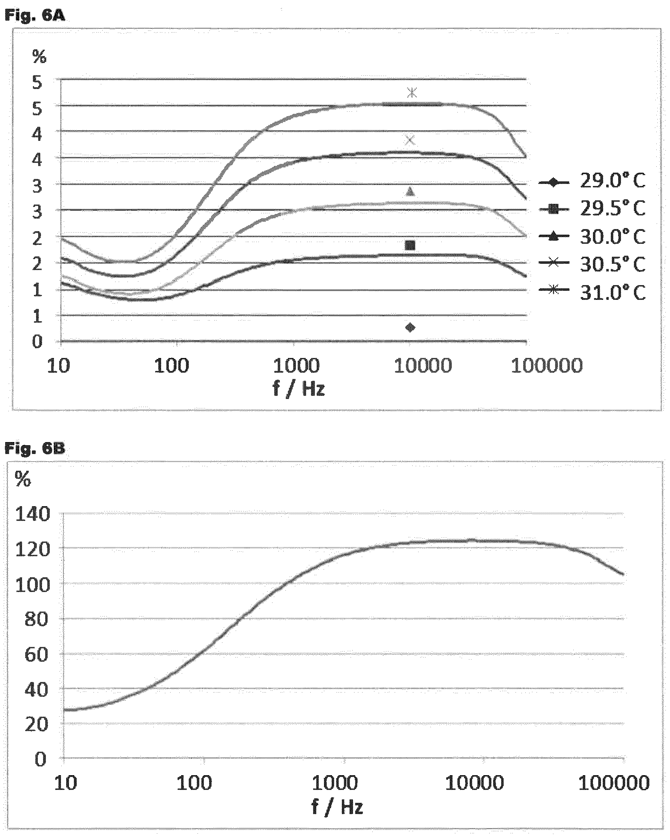

Diese Daten werden als Referenzwert (Nullprobe 1) verwendet. Dann werden Testproben mit dem gleichen Verfahren untersucht und die Ergebnisse mit den Referenzwerten verglichen. Dabei wird die prozentuale Änderung zwischen dem Nullwert und dem Probenwert normalisiert nach dem Nullwert als Koeffizient der Variation berechnet. Unterschiede, die sich erfahrungsgemäß - dies wird in einer Trainingsphase getestet - durch tageszeitliche Schwankungen, z.B. Temperaturunterschiede (Fig. 6A) oder unterschiedliche Ionenkonzentrationen (Fig. 6B) ergeben, können herausgerechnet oder ignoriert werden. Fig. Z zeigt die normalisierte Differenz (für eine Arbeitselektrode?) zwischen Trinkwasser mit und ohne Filtration. Der Unterschied weist auf eine Kontamination mit Bakterien hin.

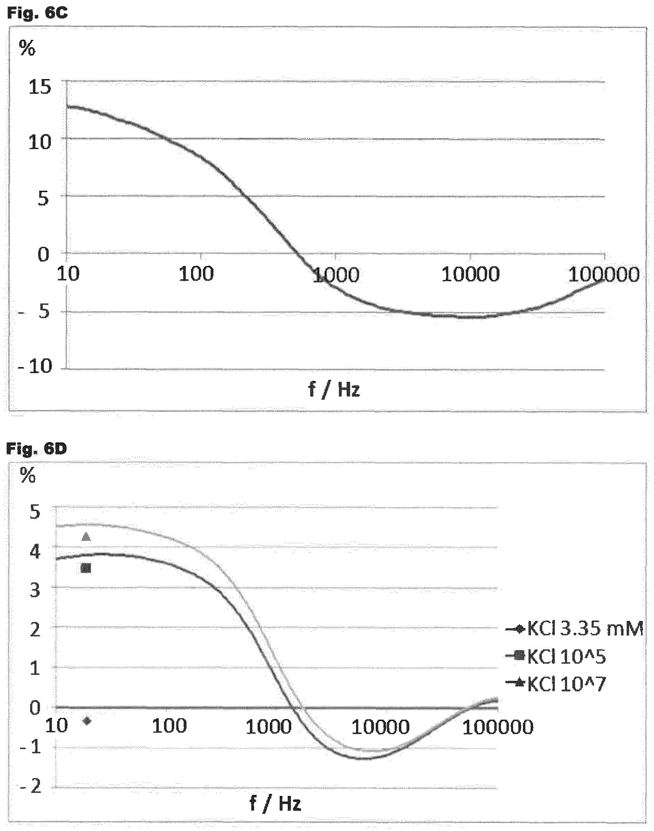

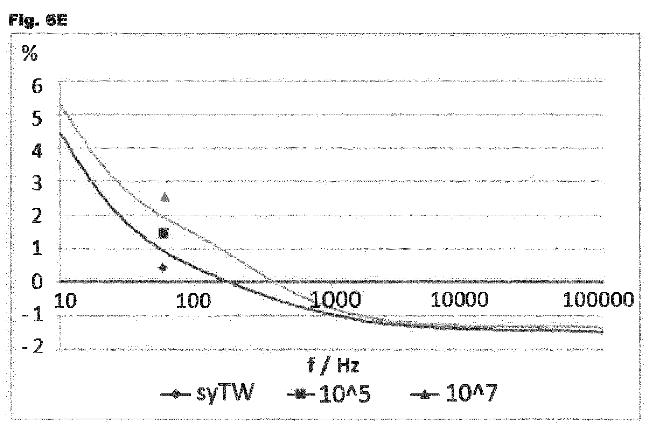

Fig. 6C und 6D zeigen die normalisierten Unterschiede zwischen Flüssigkeiten mit und ohne E. coli (Fig. 6E) bzw. mit und ohne Pseudomonas aeruginosa (Fig. 6E). Auch Saccharomyces cerevisiae konnte mit konzentrationsabhängig unterschiedlichen Signalen detektiert werden, wobei ein Nachweis ab 1 Zelle/ml möglich war.

Der Vergleich der Daten erfolgt über ein künstliches neuronales Netzwerk. Dabei wurde ein Standard-Lernverfahren verwendet, insbesondere ein resilient backprop-Lernverfahren.

Signifikante Unterschiede, welche Hinweise auf eine Kontamination mit zellulären Bestandteilen, z.B. Bakterien, liefern, lösen eine weitere Messung der gleichen Messprobe nach einem Filtervorgang, welcher die Zellen entfernt, aus, und dienen damit zur Kontrolle der Hinweise.

Ergibt sich daraus ein bestätigter Hinweis auf kritische Konzentrationen an Bakterien und/oder auf evtl. pathologisch relevante Bakterien, so kann z.B. ein Alarm ausgelöst werden. Optional wird eine Probenentnahme und Speicherung oder Versendung ausgelöst.

## Patentansprüche

1. Eine Vorrichtung zur quantitativen und/oder qualitativen Analyse von zellulären Bestandteilen einer Flüssigkeit, umfassend
a) eine Messkammer **10** umfassend ein Elektrodenarray **11** mit einer Gegenelektrode **13;**
b) ein Impedanzanalysator **20;**
**dadurch gekennzeichnet, dass** das Elektrodenarray **11** mehrere, bevorzugt mindestens zwei, sich in Bezug auf ihr Material unterscheidende Arbeitselektroden **14** umfasst.

2. Die Vorrichtung nach Anspruch 1, **dadurch gekennzeichnet, dass** die Arbeitselektroden **14** eine Oberfläche aus verschiedenen Materialien aufweisen, ausgewählt aus der Gruppe umfassend Au, Ir, Pd, Pt, Rh, Re und Fe.

3. Die Vorrichtung nach einem der vorherigen Ansprüche, **dadurch gekennzeichnet, dass** die Arbeitselektroden **14** in dem Elektrodenarray **11** konzentrisch um eine Gegenelektrode **13** angeordnet sind, wobei das Elektrodenarray **11** mindestens zwei Arbeitselektroden **14** des gleichen Materials umfasst.

4. Die Vorrichtung nach einem der vorherigen Ansprüche, **dadurch gekennzeichnet, dass** sie einen Filter **30** zum Entfernen von zellulären Bestandteilen aus der Flüssigkeit umfasst, wobei die Vorrichtung ferner bevorzugt ein Leitsystem **31** umfasst, welches es ermöglicht, die Flüssigkeit aus der Messkammer **10** durch den Filter **30** wieder in die Messkammer **10** zu leiten.

5. Die Vorrichtung nach einem der vorherigen Ansprüche, **dadurch gekennzeichnet, dass** die Arbeitselektroden **14** und die Gegenelektrode **13** sich jeweils mit einer Oberfläche **15** in Kontakt mit einer optional in der Messkammer **10** befindlichen Flüssigkeit befinden können und mit jeweils einer Oberfläche **16** mit dem Impedanzanalysator verbunden sind, während sie ansonsten durch einen Isolator **17** umschlossen sind, welcher eine Glaskeramik umfasst.

6. Die Vorrichtung nach einem der vorherigen Ansprüche, **dadurch gekennzeichnet, dass** eine online-Überwachung von zellulären Bestandteilen der Flüssigkeit möglich ist, bevorzugt eine online-Überwachung von Trinkwasser auf bakterielle zelluläre Bestandteile.

7. Die Vorrichtung nach einem der vorhergehenden Ansprüche, welche mindestens ein Mittel umfasst, dass eine Dekontamination von Messkammer **10** und, bevorzugt von Filter **30** und Leitsystem **31** erlaubt, wobei das Mittel ausgewählt ist aus der Gruppe umfassend Spüllösung, Heizung der Spüllösung, Heizung der Messkammer, Mittel zur Bestrahlung und Mittel zur Ultraschallbehandlung.

8. Ein Verfahren zur quantitativen und/oder qualitativen Analyse von zellulären Bestandteilen einer Flüssigkeit, Schritte umfassend, bei denen man
a) die Flüssigkeit in die Messkammer **10** der Vorrichtung nach einem der vorherigen Ansprüche einleitet;
b) eine Impedanzmessung durchführt;
c) eine Analyse der in Schritt b) erhaltenen Impedanzspektren durchführt.

9. Das Verfahren nach Anspruch 7, **dadurch gekennzeichnet, dass** man in Schritt b) für jede der Arbeitselektroden **14** die Impedanz für einen Frequenzbereich von 10-100 000 Hz misst, wobei man die Impedanz bevorzugt bei mindestens 192 verschiedenen Frequenzen misst.

10. Das Verfahren nach einem der Ansprüche 7 oder 8, **dadurch gekennzeichnet, dass** Schritt c) eine multivariate Datenverarbeitung umfasst, welche
i) die Impedanzwerte übernimmt;
ii) optional die Impedanzwerte filtert, um Ausreißer auszusortieren und/oder Durchschnittswerte mehrerer Messungen bildet;
iii) die erste und zweite Ableitung der Impedanzspektren oder eines Bode-Diagramms als Funktion des Betrages und/oder der Phase der Impedanz bildet,
iv) die erhaltenen Ergebnisse mit Referenzwerten vergleicht;
wobei als Ergebnis der Datenverarbeitung eine quantitative und/oder qualitative Aussage über zelluläre Bestandteile der Flüssigkeit ausgegeben wird.

11. Das Verfahren nach einem der Ansprüche 7-9, **dadurch gekennzeichnet, dass** man bei signifikanten Abweichungen der erhaltenen Impedanzwerte und/oder Ergebnisse der Datenverarbeitung zu den Referenzwerten einer Probe, welche als Nullprobe 1 bezeichnet wird
- zu analysierende Flüssigkeit von zellulären Bestandteilen befreit, bevorzugt, indem sie durch einen geeigneten Filter **30** geleitet wird, um eine Nullprobe 2 zu erhalten, und
- das Verfahren nach einem der Ansprüche 7-9 für die Nullprobe 2 durchführt,
wobei zelluläre Bestandteile vorliegen, wenn Nullprobe 1 und 2 vergleichbare Daten aufweisen.

12. Das Verfahren nach Anspruch 7-10, **dadurch gekennzeichnet, dass** man mindestens eine weitere Maßnahme durchführt, ausgewählt aus der Gruppe umfassend
a) Lyse zellulärer Bestandteile und Bestimmung von Impedanzwerten vor und nach Lyse;
b) eine weitere elektrochemische, biochemische oder physikalische Messung, ausgewählt aus der Gruppe umfassend Temperaturmessung, Leitfähigkeitsmessung, pH-Messung, Antikörperbasiertes Assay;
c) Speicherung oder Veranlassung der Entnahme einer Probe der Flüssigkeit bei Feststellung von zellulären Bestandteilen.

13. Das Verfahren nach Anspruch 7-11, **dadurch gekennzeichnet, dass** eine online-Überwachung von zellulären Bestandteilen der Flüssigkeit durchgeführt wird, bevorzugt eine online-Überwachung von Trinkwasser auf bakterielle zelluläre Bestandteile.

14. Die Vorrichtung nach einem der Ansprüche 1-6, **dadurch gekennzeichnet, dass** sie Mittel zur Steuerung und Auswertung des Verfahrens nach einem der Ansprüche 7-12 umfasst, wobei die Mittel bevorzugt einen Computer mit einem Computerprogrammprodukt zur Steuerung und Auswertung des Verfahrens nach einem der Ansprüche 7-12 umfassen.

15. Die Vorrichtung nach einem der Ansprüche 1-6 oder 13 oder das Verfahren nach einem der Ansprüche 7-12, **dadurch gekennzeichnet, dass** die Flüssigkeit ausgewählt ist aus der Gruppe umfassend Trinkwasser, Betriebswasser, Oberflächenwasser, Abwasser, Getränke, Zwischenprodukte der Getränkeherstellung, Zellkulturmedium und Zellkultur, bevorzugt Wasser, am meisten bevorzugt Trinkwasser.

16. Die Vorrichtung nach einem der Ansprüche 1-6 oder 13-14 oder das Verfahren nach einem der Ansprüche 7-12 oder 14, **dadurch gekennzeichnet, dass** die zellulären Bestandteile 1 ausgewählt sind aus der Gruppe umfassend Bakterien, Hefen und andere eukaryontische Zellen, bevorzugt Bakterien.

17. Computerprogrammprodukt, welches in den internen Speicher eines Computers geladen werden kann oder auf einem computerlesbaren Speichermedium gespeichert ist, umfassend computerlesbare Programm-Mittel zur Steuerung der Vorrichtung nach einem der Ansprüche 1-6 oder 13-15 bei der Durchführung des Verfahrens nach einem der Ansprüche 7-12 oder 14-15 oder bei der Durchführung der Analyse des Verfahrens nach einem der Ansprüche 7-12 oder 14-15, wenn das Computerprogrammprodukt auf einem Computer ausgeführt wird, wobei die Analyse bevorzugt die Simulation eines neuronalen Netzwerkes umfasst.

18. Verwendung der Vorrichtung nach einem der Ansprüche 1-6 oder 13-15, des Verfahrens nach einem der Ansprüche 7-12 oder 14-15 oder des Computerprogrammprodukts nach Anspruch 16 zur quantitativen und/oder qualitativen Analyse von zellulären Bestandteilen einer Flüssigkeit, bevorzugt zur quantitativen und qualitativen Analyse von Trinkwasser auf bakterielle zelluläre Bestandteile.
